# EUROPEAN PATENT SPECIFICATION

(11) **EP 1 241 254 B1**
(45) Date of publication and mention of the grant of the patent: **20.07.2005**
(21) Application number: 00979978.4
(22) Date of filing: 07.12.2000
(51) Int. Cl.: C12N 15/12, C07K 14/82, A61K 38/17, C12N 15/18, A61K 31/711, A61K 48/00, A61P 43/00, A61P 25/28, A61P 21/04, A61P 9/10, A61P 1/16

(54) **APOPTOSIS INHIBITORY POLYPEPTIDE, GENE AND POLYNUCLEOTIDE ENCODING IT AND COMPOSITIONS CONTAINING THE SAME**
APOPTOSE-HEMMENDES POLYPEPTID, FüR DIESES KODIERENDE GENE UND POLYNUKLEOTIDE UND DIESE BEINHALTENDE ZUSAMMENSETZUNGEN
POLYPEPTIDE INHIBITEUR D'APOPTOSE, GENE ET POLYNUCLEOTIDE LE CODANT ET COMPOSITIONS LE CONTENANT

(30) Priority: 09.12.1999 JP 35042799
(43) Date of publication of application: 18.09.2002
(73) Proprietor: HISAMITSU PHARMACEUTICAL CO. INC., Tosu-shi, Saga-ken 841-0017 (JP)
(72) Inventor: SHIBAZAKI, Futoshi The Tokyo Metropolitan Institut, Tokyo 113-0021 (JP); KUMA, Hidekazu Tsukuba Lab. Hisamitsu Phar. Co.Inc, Ibaraki 305-0856 (JP)
(74) Representative: Grünecker, Kinkeldey, Stockmair & Schwanhäusser Anwaltssozietät
(86) International application number: PCT/JP2000/008667
(87) International publication number: WO 2001/042459

(56) References cited:
- YAMAMOTO KAZUHITO ET AL: "BCL-2 is phosphorylated and inactivated by an ASK1/Jun N-terminal protein kinase pathway normally activated at G2/M" MOLECULAR AND CELLULAR BIOLOGY, vol. 19, no. 12, December 1999 (1999-12), pages 8469-8478, XP002268035 ISSN: 0270-7306
- RUVOLO PETER P ET AL: "Ceramide induces Bcl2 dephosphorylation via a mechanism involving mitochondrial PP2A" JOURNAL OF BIOLOGICAL CHEMISTRY, vol. 274, no. 29, 16 July 1999 (1999-07-16), pages 20296-20300, XP002268036 ISSN: 0021-9258
- SHIBASAKI F. ET AL.: 'Suppression of signalling through transcription factor NF-AT by interactions between calcineurin and Bcl-2' NATURE vol. 386, no. 6626, 1997, pages 728 - 731, XP002936733
- HALDAR S. ET AL.: 'Inactivation of Bcl-2 by phosphorylation' PROC. NATL. ACAD. SCI. USA vol. 92, no. 10, 1995, pages 4507 - 4511, XP002936734
- DANIELLE DE MOISSAC ET AL.: 'Linkage of the BH4 domain of Bcl-2 and the nuclear factor kappaB signaling pathway for suppression of apoptosis' J. BIOL. CHEM. vol. 274, no. 41, October 1999, pages 29505 - 29509, XP002936735
- HIROTANI M. ET AL.: 'NH2-terminal BH4 domain of Bcl-2 is functional for heterodimerization with Bax and inhibition of apoptosis' J. BIOL. CHEM. vol. 274, no. 29, July 1999, pages 20415 - 20420, XP002936736
- ITO T. ET AL.: 'Bcl-2 phosphorylation required for anti-apoptosis function' J. BIOL. CHEM. vol. 272, no. 18, 1997, pages 11671 - 11673, XP002936737

### SEQUENCE LISTING

<110> Hisamitsu Pharmaceuticals Co., Inc.
<120> Apoptosis-inhibiting polypeptides, genes and polynucleotides encoding same, and compositions containing them
<130> EP 24654-031/GRI
<150> JP 11/350427
   <151> 1999-12-09
<160> 21
<170> PatentIn Ver. 2.0
<210> 1
   <211> 239
   <212> PRT
   <213> Human
<400> 1
<210> 2
   <211> 239
   <212> PRT
   <213> Artificial Sequence
<220>
   <223> Description of Artificial Sequence: Variant of the wild type Bcl-2 protein
<400> 2
   Met Ala His Ala Gly Arg Thr Gly Tyr Asp Asn Arg Glu Ile Val Met
   1 5 10 15
   Lys Tyr Ile His Tyr Lys Leu Ala Gln Arg Gly Tyr Glu Trp Asp Ala
   20 25 30
   Gly Asp Val Gly Ala Ala Pro Pro Gly Ala Ala Pro Ala Pro Gly Ile
   35 40 45
   Phe Ser Ser Gln Pro Gly His Thr Pro His Pro Ala Ala Ser Arg Asp
   50 55 60
   Pro Val Ala Arg Thr Ser Pro Leu Gln Thr Pro Ala Ala Pro Gly Ala
   65 70 75 80
   Ala Ala Gly Pro Ala Leu Ser Pro Val Pro Pro Val Val His Leu Ala
   85 90 95
   Leu Arg Gln Ala Gly Asp Asp Phe Ser Arg Arg Tyr Arg Gly Asp Phe
   100 105 110
   Ala Glu Met Ser Ser Gln Leu His Leu Thr Pro Phe Thr Ala Arg Gly
   115 120 125
   Arg Phe Ala Thr Val Val Glu Glu Leu Phe Arg Asp Gly Val Asn Trp
   130 135 140
   Gly Arg Ile Val Ala Phe Phe Glu Phe Gly Gly Val Met Cys Val Glu
   145 150 155 160
   Ser Val Asn Arg Glu Met Ser Pro Leu Val Asp Asn Ile Ala Leu Trp
   165 170 175
   Met Thr Glu Tyr Leu Asn Arg His Leu His Thr Trp Ile Gln Asp Asn
   180 185 190
   Gly Gly Trp Asp Ala Phe Val Glu Leu Tyr Gly Pro Ser Met Arg Pro
   195 200 205
   Leu Phe Asp Phe Ser Trp Leu Ser Leu Lys Thr Leu Leu Ser Leu Ala
   210 215 220
   Leu Val Gly Ala Cys Ile Thr Leu Gly Ala Tyr Leu Ser His Lys
   225 230 235
<210> 3
   <211> 239
   <212> PRT
   <213> Artificial Sequence
<220>
   <223> Description of Artificial Sequence: Variant of the wild type Bcl-2 protein
<400> 3
   Met Ala His Ala Gly Arg Thr Gly Tyr Asp Asn Arg Glu Ile Val Met
   1 5 10 15
   Lys Tyr Ile His Tyr Lys Leu Ser Gln Arg Gly Tyr Glu Trp Asp Ala
   20 25 30
   Gly Asp Val Gly Ala Ala Pro Pro Gly Ala Ala Pro Ala Pro Gly Ile
   35 40 45
   Phe Ser Ser Gln Pro Gly His Thr Pro His Pro Ala Ala Ser Arg Asp
   50 55 60
   Pro Val Ala Arg Thr Ser Pro Leu Gln Thr Pro Ala Ala Pro Gly Ala
   65 70 75 80
   Ala Ala Gly Pro Ala Leu Ser Pro Val Pro Pro Val Val His Leu Ala
   85 90 95
   Leu Arg Gln Ala Gly Asp Asp Phe Ser Arg Arg Tyr Arg Gly Asp Phe
   100 105 110
   Ala Glu Met Ala Ala Gln Leu His Leu Thr Pro Phe Thr Ala Arg Gly
   115 120 125
   Arg Phe Ala Thr Val Val Glu Glu Leu Phe Arg Asp Gly Val Asn Trp
   130 135 140
   Gly Arg Ile Val Ala Phe Phe Glu Phe Gly Gly Val Met Cys Val Glu
   145 150 155 160
   Ser Val Asn Arg Glu Met Ser Pro Leu Val Asp Asn Ile Ala Leu Trp
   165 170 175
   Met Thr Glu Tyr Leu Asn Arg His Leu His Thr Trp Ile Gln Asp Asn
   180 185 190
   Gly Gly Trp Asp Ala Phe Val Glu Leu Tyr Gly Pro Ser Met Arg Pro
   195 200 205
   Leu Phe Asp Phe Ser Trp Leu Ser Leu Lys Thr Leu Leu Ser Leu Ala
   210 215 220
   Leu Val Gly Ala Cys Ile Thr Leu Gly Ala Tyr Leu Ser His Lys
   225 230 235
<210> 4
   <211> 239
   <212> PRT
   <213> Artificial Sequence
<220>
   <223> Description of Artificial Sequence: Variant of the wild type Bcl-2 protein
<400> 4
   Met Ala His Ala Gly Arg Thr Gly Tyr Asp Asn Arg Glu Ile Val Met
   1 5 10 15
   Lys Tyr Ile His Tyr Lys Leu Ser Gln Arg Gly Tyr Glu Trp Asp Ala
   20 25 30
   Gly Asp Val Gly Ala Ala Pro Pro Gly Ala Ala Pro Ala Pro Gly Ile
   35 40 45
   Phe Ser Ser Gln Pro Gly His Thr Pro His Pro Ala Ala Ser Arg Asp
   50 55 60
   Pro Val Ala Arg Thr Ser Pro Leu Gln Thr Pro Ala Ala Pro Gly Ala
   65 70 75 80
   Ala Ala Gly Pro Ala Leu Ser Pro Val Pro Pro Val Val His Leu Ala
   85 90 95
   Leu Arg Gln Ala Gly Asp Asp Phe Ser Arg Arg Tyr Arg Gly Asp Phe
   100 105 110
   Ala Glu Met Ser Ser Gln Leu His Leu Thr Pro Phe Thr Ala Arg Gly
   115 120 125
   Arg Phe Ala Thr Val Val Glu Glu Leu Phe Arg Asp Gly Val Asn Trp
   130 135 140
   Gly Arg Ile Val Ala Phe Phe Glu Phe Gly Gly Val Met Cys Val Glu
   145 150 155 160
   Ala Val Asn Arg Glu Met Ser Pro Leu Val Asp Asn Ile Ala Leu Trp
   165 170 175
   Met Thr Glu Tyr Leu Asn Arg His Leu His Thr Trp Ile Gln Asp Asn
   180 185 190
   Gly Gly Trp Asp Ala Phe Val Glu Leu Tyr Gly Pro Ser Met Arg Pro
   195 200 205
   Leu Phe Asp Phe Ser Trp Leu Ser Leu Lys Thr Leu Leu Ser Leu Ala
   210 215 220
   Leu Val Gly Ala Cys Ile Thr Leu Gly Ala Tyr Leu Ser His Lys
   225 230 235
<210> 5
   <211> 239
   <212> PRT
   <213> Artificial Sequence
<220>
   <223> Description of Artificial Sequence: Variant of the wild type Bcl-2 protein
<400> 5
   Met Ala His Ala Gly Arg Thr Gly Tyr Asp Asn Arg Glu Ile Val Met
   1 5 10 15
   Lys Tyr Ile His Tyr Lys Leu Ser Gln Arg Gly Tyr Glu Trp Asp Ala
   20 25 30
   Gly Asp Val Gly Ala Ala Pro Pro Gly Ala Ala Pro Ala Pro Gly Ile
   35 40 45
   Phe Ser Ser Gln Pro Gly His Thr Pro His Pro Ala Ala Ser Arg Asp
   50 55 60
   Pro Val Ala Arg Thr Ser Pro Leu Gln Thr Pro Ala Ala Pro Gly Ala
   65 70 75 80
   Ala Ala Gly Pro Ala Leu Ser Pro Val Pro Pro Val Val His Leu Ala
   85 90 95
   Leu Arg Gln Ala Gly Asp Asp Phe Ser Arg Arg Tyr Arg Gly Asp Phe
   100 105 110
   Ala Glu Met Ser Ser Gln Leu His Leu Thr Pro Phe Thr Ala Arg Gly
   115 120 125
   Arg Phe Ala Thr Val Val Glu Glu Leu Phe Arg Asp Gly Val Asn Trp
   130 135 140
   Gly Arg Ile Val Ala Phe Phe Glu Phe Gly Gly Val Met Cys Val Glu
   145 150 155 160
   Asp Val Asn Arg Glu Met Ser Pro Leu Val Asp Asn Ile Ala Leu Trp
   165 170 175
   Met Thr Glu Tyr Leu Asn Arg His Leu His Thr Trp Ile Gln Asp Asn
   180 185 190
   Gly Gly Trp Asp Ala Phe Val Glu Leu Tyr Gly Pro Ser Met Arg Pro
   195 200 205
   Leu Phe Asp Phe Ser Trp Leu Ser Leu Lys Thr Leu Leu Ser Leu Ala
   210 215 220
   Leu Val Gly Ala Cys Ile Thr Leu Gly Ala Tyr Leu Ser His Lys
   225 230 235
<210> 6
   <211> 720
   <212> DNA
   <213> Human
<400> 6
   atggcgcacg ctgggagaac ggggtacgac aaccgggaga tagtgatgaa gtacatccat 60
   tataagctgt cgcagagggg ctacgagtgg gatgcgggag atgtgggcgc cgcgcccccg 120
   ggggccgccc ccgcaccggg catcttctcc tcccagcccg ggcacacgcc ccatccagcc 180
   gcatcccgcg acccggtcgc caggacctcg ccgctgcaga ccccggctgc ccccggcgcc 240
   gccgcggggc ctgcgctcag cccggtgcca cctgtggtcc acctggccct ccgccaagcc 300
   ggcgacgact tctcccgccg ctaccgcggc gacttcgccg agatgtccag ccagctgcac 360
   ctgacgccct tcaccgcgcg gggacgcttt gccacggtgg tggaggagct cttcagggac 420
   ggggtgaact gggggaggat tgtggccttc tttgagttcg gtggggtcat gtgtgtggag 480
   agcgtcaacc gggagatgtc gcccctggtg gacaacatcg ccctgtggat gactgagtac 540
   ctgaaccggc acctgcacac ctggatccag gataacggag gctgggatgc ctttgtggaa 600
   ctgtacggcc ccagcatgcg gcctctgttt gatttctcct ggctgtctct gaagactctg 660
   ctcagtttgg ccctggtggg agcttgcatc accctgggtg cctatctgag ccacaagtga 720
<210> 7
   <211> 720
   <212> DNA
   <213> Artificial Sequence
<220>
   <223> Description of Artificial Sequence: Nucleotide Variant of the wild type Bcl-2 gene
<400> 7
   atggcgcacg ctgggagaac ggggtacgac aaccgggaga tagtgatgaa gtacatccat 60
   tataagctgg cgcagagggg ctacgagtgg gatgcgggag atgtgggcgc cgcgcccccg 120
   ggggccgccc ccgcaccggg catcttctcc tcccagcccg ggcacacgcc ccatccagcc 180
   gcatcccgcg acccggtcgc caggacctcg ccgctgcaga ccccggctgc ccccggcgcc 240
   gccgcggggc ctgcgctcag cccggtgcca cctgtggtcc acctggccct ccgccaagcc 300
   ggcgacgact tctcccgccg ctaccgcggc gacttcgccg agatgtccag ccagctgcac 360
   ctgacgccct tcaccgcgcg gggacgcttt gccacggtgg tggaggagct cttcagggac 420
   ggggtgaact gggggaggat tgtggccttc tttgagttcg gtggggtcat gtgtgtggag 480
   agcgtcaacc gggagatgtc gcccctggtg gacaacatcg ccctgtggat gactgagtac 540
   ctgaaccggc acctgcacac ctggatccag gataacggag gctgggatgc ctttgtggaa 600
   ctgtacggcc ccagcatgcg gcctctgttt gatttctcct ggctgtctct gaagactctg 660
   ctcagtttgg ccctggtggg agcttgcatc accctgggtg cctatctgag ccacaagtga 720
<210> 8
   <211> 720
   <212> DNA
   <213> Artificial Sequence
<220>
   <223> Description of Artificial Sequence: Nucleotide Variant of the wild type Bcl-2 gene
<400> 8
   atggcgcacg ctgggagaac ggggtacgac aaccgggaga tagtgatgaa gtacatccat 60
   tataagctgt cgcagagggg ctacgagtgg gatgcgggag atgtgggcgc cgcgcccccg 120
   ggggccgccc ccgcaccggg catcttctcc tcccagcccg ggcacacgcc ccatccagcc 180
   gcatcccgcg acccggtcgc caggacctcg ccgctgcaga ccccggctgc ccccggcgcc 240
   gccgcggggc ctgcgctcag cccggtgcca cctgtggtcc acctggccct ccgccaagcc 300
   ggcgacgact tctcccgccg ctaccgcggc gacttcgccg agatggccgc ccagctgcac 360
   ctgacgccct tcaccgcgcg gggacgcttt gccacggtgg tggaggagct cttcagggac 420
   ggggtgaact gggggaggat tgtggccttc tttgagttcg gtggggtcat gtgtgtggag 480
   agcgtcaacc gggagatgtc gcccctggtg gacaacatcg ccctgtggat gactgagtac 540
   ctgaaccggc acctgcacac ctggatccag gataacggag gctgggatgc ctttgtggaa 600
   ctgtacggcc ccagcatgcg gcctctgttt gatttctcct ggctgtctct gaagactctg 660
   ctcagtttgg ccctggtggg agcttgcatc accctgggtg cctatctgag ccacaagtga 720
<210> 9
   <211> 720
   <212> DNA
   <213> Artificial Sequence
<220>
   <223> Description of Artificial Sequence: Nucleotide Variant of the wild type Bcl-2 gene
<400> 9
   atggcgcacg ctgggagaac ggggtacgac aaccgggaga tagtgatgaa gtacatccat 60
   tataagctgt cgcagagggg ctacgagtgg gatgcgggag atgtgggcgc cgcgcccccg 120
   ggggccgccc ccgcaccggg catcttctcc tcccagcccg ggcacacgcc ccatccagcc 180
   gcatcccgcg acccggtcgc caggacctcg ccgctgcaga ccccggctgc ccccggcgcc 240
   gccgcggggc ctgcgctcag cccggtgcca cctgtggtcc acctggccct ccgccaagcc 300
   ggcgacgact tctcccgccg ctaccgcggc gacttcgccg agatgtccag ccagctgcac 360
   ctgacgccct tcaccgcgcg gggacgcttt gccacggtgg tggaggagct cttcagggac 420
   ggggtgaact gggggaggat tgtggccttc tttgagttcg gtggggtcat gtgtgtggag 480
   gccgtcaacc gggagatgtc gcccctggtg gacaacatcg ccctgtggat gactgagtac 540
   ctgaaccggc acctgcacac ctggatccag gataacggag gctgggatgc ctttgtggaa 600
   ctgtacggcc ccagcatgcg gcctctgttt gatttctcct ggctgtctct gaagactctg 660
   ctcagtttgg ccctggtggg agcttgcatc accctgggtg cctatctgag ccacaagtga 720
<210> 10
   <211> 720
   <212> DNA
   <213> Artificial Sequence
<220>
   <223> Description of Artificial Sequence: Nucleotide Variant of the wild type Bcl-2 gene
<400> 10
   atggcgcacg ctgggagaac ggggtacgac aaccgggaga tagtgatgaa gtacatccat 60
   tataagctgt cgcagagggg ctacgagtgg gatgcgggag atgtgggcgc cgcgcccccg 120
   ggggccgccc ccgcaccggg catcttctcc tcccagcccg ggcacacgcc ccatccagcc 180
   gcatcccgcg acccggtcgc caggacctcg ccgctgcaga ccccggctgc ccccggcgcc 240
   gccgcggggc ctgcgctcag cccggtgcca cctgtggtcc acctggccct ccgccaagcc 300
   ggcgacgact tctcccgccg ctaccgcggc gacttcgccg agatgtccag ccagctgcac 360
   ctgacgccct tcaccgcgcg gggacgcttt gccacggtgg tggaggagct cttcagggac 420
   ggggtgaact gggggaggat tgtggccttc tttgagttcg gtggggtcat gtgtgtggag 480
   gacgtcaacc gggagatgtc gcccctggtg gacaacatcg ccctgtggat gactgagtac 540
   ctgaaccggc acctgcacac ctggatccag gataacggag gctgggatgc ctttgtggaa 600
   ctgtacggcc ccagcatgcg gcctctgttt gatttctcct ggctgtctct gaagactctg 660
   ctcagtttgg ccctggtggg agcttgcatc accctgggtg cctatctgag ccacaagtga 720
<210> 11
   <211> 27
   <212> DNA
   <213> Artificial Sequence
<220>
   <223> Description of Artificial Sequence: Oligonucleotide for introducing S24A variation
<400> 11
   gtagccggtc tgcgccagct tataatg 27
<210> 12
   <211> 27
   <212> DNA
   <213> Artificial Sequence
<220>
   <223> Description of Artificial Sequence: Oligonucleotide for introducing S24D variation
<400> 12
   gtagccggtc tggtccagct tataatg 27
<210> 13
   <211> 27
   <212> DNA
   <213> Artificial Sequence
<220>
   <223> Description of Artificial Sequence: Oligonucleotide for introducing S70A variation
<400> 13
   ggtctgcagc ggcgcggtcc tggcgac 27
<210> 14
   <211> 27
   <212> DNA
   <213> Artificial Sequence
<220>
   <223> Description of Artificial Sequence: Oligonucleotide for introducing S70D variation
<400> 14
   ggtctgcagc gggtcggtcc tggcgac 27
<210> 15
   <211> 27
   <212> DNA
   <213> Artificial Sequence
<220>
   <223> Description of Artificial Sequence: Oligonucleotide for introducing S116A variation
<400> 15
   gtgcagctgg ctggccatct cggcgaa 27
<210> 16
   <211> 27
   <212> DNA
   <213> Artificial Sequence
<220>
   <223> Description of Artificial Sequence: Oligonucleotide for introducing S116D variation
<400> 16
   gtgcagctgg ctgaccatct cggcgaa 27
<210> 17
   <211> 27
   <212> DNA
   <213> Artificial Sequence
<220>
   <223> Description of Artificial Sequence: Oligonucleotide for introducing S116A/S117A variation
<400> 17
   gtgcagctgg gcggccatct cggcgaa 27
<210> 18
   <211> 27
   <212> DNA
   <213> Artificial Sequence
<220>
   <223> Description of Artificial Sequence: Oligonucleotide for introducing S161A variation
<400> 18
   ctcccggttg acggcctcca cacacat 27
<210> 19
   <211> 27
   <212> DNA
   <213> Artificial Sequence
<220>
   <223> Description of Artificial Sequence: Oligonucleotide for introducing S161D variation
<400> 19
   ctcccggttg acgacctcca cacacat 27
<210> 20
   <211> 36
   <212> DNA
   <213> Artificial Sequence
<220>
   <223> Description of Artificial Sequence: Primer
<400> 20
   ccgctcgaga tggcgcacgc tgggagaacg gggtac 36
<210> 21
   <211> 32
   <212> DNA
   <213> Artificial Sequence
<220>
   <223> Description of Artificial Sequence: Primer
<400> 21
   gctctagatc acttgtggct cagataggca cc 32

## Description

### Technical Field

This invention relates to a gene regulating the apoptosis of cells. More particularly, the invention relates to a variant of oncogene Bcl-2 derived from human follicular B cell lymphoma and to a variant of the apoptosis inhibitory Bcl-2 protein encoded by the former variant.

### Background Art

### 1. Cell Death and Apoptosis

In the past, for cell death, only the "necrosis" was known which was the pathological cell death where a group of cells died of burn or the like. In 1972, the three pathologists, Kerr, Wyllie and Currie discovered apoptosis and defined that it was the cell death which differs from necrosis in morphological aspects (Kerr et al., Br, J. Cancer 26, 239 (1972)). It was proposed that apoptosis was different from necrosis not only in morphology, but also in function and that apoptosis had broad significance in the cell kinetics of tissue. Because of its characteristics, it has been regarded as the active process programmed in genes (programmed cell death).

Apoptosis is a process by which many cells die for purposes of the growth and maintenance of the cells themselves in complex eucaryotes. The cell death by apoptosis occurs when the cell activates its suicide program coded within through exogenous or endogenous signals. Apoptosis is characterized by the blebbing of cell membrane, the loss in cell volume, karyopyknosis (nuclear condensation) and the decomposition of DNA at nucleosome spaces (Wyllie et al., Int. Rev. Cytol. 68, 251 (1980)).

Apoptosis plays an indispensable role in the maintenance of function of the normal body from the early phase of the birth of life beginning with one fertilized egg to death; and its aberration is involved in a number of diseases including carcinoma. It has been revealed that common genes participate in the mechanism of such cell death. In addition, it is beginning to be understood that many of oncogenes and cancer-suppressing genes are related to apoptosis. Apoptosis is also seen in the manifestation of physiological phenomena such as immunity and hormonal action and it plays an important role as "physiological cell death" which is essential to these life phenomena. Recent studies on the mechanism for regulating the programmed cell death are advancing very rapidly (Williams, Cell 65, 1097 (1991)).

### 2. Aberration of Apoptosis, Disease and Therapy

The immune system central to biological defense mechanism is the living system that is most closely related to apoptosis. In the development of the immune system centered on the thymus, the aberration of apoptosis-associated genes causes an autoimmune disease. In viral infections such as HIV, apoptosis may induce abrupt reduction in lymphocytes. Apoptosis also plays an important role in the development of nervous system. In addition, apoptosis is regarded as important in the disorders involving nerve cell death such as Alzheimer disease, Parkinson disease, and dysmnesia resulting from reperfusion damage. Involvement of apoptosis in the pathology of carcinogenesis has also caught much attention. As compared with the high frequency of carcinogenesis in the large intestine, the frequency of carcinogenesis in the small intestine is extremely low: the high occurrence of apoptosis in the intestinum tenue epithelia stem cell accounts for it. Thus, the possibility can be suggested that apoptosis is directly involved in the pathology of carcinogenesis.

In addition to the etiologic investigation as described above, a new thought is going to be created in the field of therapy. So far only the growth inhibition of cancer cells has been the target of treatment in cancer therapy, but an approach is coming out that the apoptosis of the cancer cells is actively induced to enhance therapeutic effects.

In this way, since too much or too little in cell death is involved in many diseases, expectation in the therapy by the control of apoptosis is rising. Accordingly, the possibility will be opened up that the development of factors or drugs which promote or inhibit apoptosis can be applied to the therapy of diseases associated with apoptosis.

### 3. Apoptosis-Associated Genes (Bcl-2 gene)

In 1985 Tsujimoto et al. discovered Bcl-2 gene as an oncogene that was located in the neighborhood of the t (14; 18) (q32; q21) translocation point which was found with high frequency in human follicular B cell lymphoma (Tsujimoto Y. et al, Science 228, 1440 (1985)). Since Vaux et al. reported the apoptosis inhibitory effect of Bcl-2 in 1988, the protective effect of Bcl-2 has been shown in many systems where apoptosis is induced. Searches for the genes homologous to the Bcl-2 as well as for factors binding to the Bcl-2 protein have been enthusiastically conducted, and a large number of family genes have been identified since 1993.

The Bcl-2 gene consists of three exons. The Bcl-2 gene generates plural mRNAs depending on the presence of splicing, and the production of two kinds of proteins (Bcl-2α with 26kD and Bcl-2β with 22kD) is expected; however, only Bcl-2α has been detected in the living body. The Bcl-2 protein has a hydrophobic amino acid domain at its C-terminal which serves as a membrane localization signal and is present in extranuclear membrane, endoplasmic reticulum membrane, and mitochondrial membrane.

It has been shown that Bcl-2 can efficiently inhibit the apoptosis induced by almost any stimuli and it is thought that the Bcl-2 functions in the common path of apoptosis with Bcl-XL. On the other hand, Bax, Bak, Bad, Bik, and Bcl-XS, which are other members of the Bcl-2 family, suppress the inhibitory effect of Bcl-2 or Bcl-XL. It is thought that this function of Bax and the others can be achieved by forming a heterodimer with Bcl-2 or with Bcl-XL. It has been reported that Bcl-2 can bind to R-Ras, Bag-1, and Raf-1 besides the family members mentioned above. In addition, Bcl-2 and Bcl-XL can suppress some necrosis (through mitochondrial respiratory chain inhibition).

The transgenic mouse that overexpresses the Bcl-2 in the B cell system exhibits benign lymphadenopathy and then develops a malignant lymphoma with constant frequency; in addition, the crisis of an autoimmune disease resembling SLE (systemic lupus erythematodes) is observed. The transgenic mouse that overexpresses the Bcl-2 in T cells does not exhibit excessive T cell accumulation. It is shown that the removal of autoantigen recognition T cells from T cells occurred in the thymus progresses almost normally.

In the transgenic mouse where a high level expression of Bcl-2 in nerve cells is caused, the removal of the excessive nerve cells generated during the normal ontogenesis is suppressed. This mouse also shows resistance to cell death by denervation or ischemia. In the transgenic mouse where a high level expression of Bcl-2 in hepatic cells is caused, the induction of fulminant hepatitis by anti-Fas antibody is significantly suppressed.

A Bcl-2 defect mouse is born normally at a glance, but is accompanied by aberration due to a short cycle of lymphocytes and intestinal epithelium in addition to aberration such as growth retardation, little external ear pinna, canities, and multicystic kidney. In thymus T cells, the expression of Bcl-2 is observed in a mature T cell (CD4+CD8- and CD4-CD8+), and its expression in an immature T cell (CD4+CD8) is low. It is shown that the Bcl-2 plays an important role in the maintenance of mature thymocytes.

### 4. Phosphorylation of the Bcl-2 Gene

Haldar et al. pointed out the possibility that the Bcl-2 lost its apoptosis inhibitory effect through Bcl-2 phosphorylation (Haldar S. et al., PNAS 92, 4507 (1995)). They found that part of serine residues in the Bcl-2 underwent phosphorylation in lymphocytes and showed that the anti-apoptosis ability of the Bcl-2 was inhibited in the system okadaic acid and taxol were added which were hydrolase inhibitors of phosphoric acid.

Ito et al. (J. Biol. Chem. 1997, Vol. 272, pp. 11671-11673) disclosed that Bcl-2 phosphorylation is required for full Bcl-2 death suppressor signaling activity. By producing conservative Ser → Ala or Ser → Glu mutations at the seven putative protein kinase C phosphorylation sites in murine Bcl-2, it was found that S70E showed apoptosis inhibitory activity but S70A showed pro-apoptotic acticity.

In addition, Shibasaki found that calcineurin, which is a dephosphorylation enzyme of serine/threonine bound to the BH-4 domain (Shibasaki F. et al., Nature 386, 728-731 (1997)). They showed that calcineurin, which is a dephosphorylation enzyme of serine/threonine regulated phosphorylation/dephosphorylation and controlled the function of the Bcl-2 by directly binding to the BH-4 domain of Bcl-2.

### Disclosure of the Invention

This invention aims at providing a Bcl-2 variant protein possessing more potent apoptosis inhibitory activity than does the wild type Bcl-2, as well as providing a Bcl-2 variant gene encoding the protein.

As stated above, it is anticipated that the phosphorylation/dephosphorylation of serine residues in the Bcl-2 molecule influences the apoptosis inhibitory activity. Accordingly, the present inventors focused the importance of these serine residues and carried out studies diligently to improve the apoptosis inhibitory activity of the wild type Bcl-2.

The present inventors also discovered that the variant where alanine substituted for a particular position(s) existing other than in the BH-4 domain displayed significantly enhanced apoptosis inhibitory activity as compared with the wild type Bcl-2; and this invention was thus accomplished.

In other words, the gist of this invention is that by substituting alanine for serine among amino acids constituting the wild type Bcl-2 protein to enhance its apoptosis inhibitory activity, the resulting variant protein or the variant gene encoding them are used for the treatment of various diseases in which apoptosis is involved and the therapeutic effects are to be achieved based on the enhanced apoptosis inhibitory effect.

Consequently, there is provided according to this invention a polypeptide comprising an amino acid sequence derivable from the substitution of two serines by alanine in the amino sequence set forth in SEQ ID NO:1 in the Sequence Listing, said polypeptide possessing apoptosis inhibitory activity.

Preferably, there is provided according to this invention a polypeptide comprising an amino acid sequence derivable from the substitution of two serines by alanine in the amino sequence set forth in SEQ ID NO:1 in the Sequence Listing, said polypeptide possessing apoptosis inhibitory activity substantially higher than that of the wild type Bcl-2 protein.

More preferably, the invention is characterized in that the 116th and the 117th serine is substituted by alanine in the polypeptide mentioned above.

Specifically, this invention provides the protein described below: A polypeptide comprising the amino acid sequence set forth in SEQ ID NO:3 in the Sequence Listing.

The polypeptide described above is a variant of the wild type Bcl-2 protein and is provided with the apoptosis inhibitory activity substantially higher that that of said protein.

Furthermore, this invention provides a pharmaceutical composition comprising the polypeptide described above possessing the apoptosis inhibitory activity as describe above together with a pharmaceutically acceptable carrier or diluent. Here, the polypeptide may be in the form of its respective pharmaceutically acceptable salts.

This invention also provides a polynucleotide which encodes the aforementioned polypeptide possessing the apoptosis inhibitory activity.

More specifically, this invention provides the polynucleotide described below: A polynucleotide comprising the nucleotide sequence set forth in SEQ ID NO:8 in the Sequence Listing.

The polynucleotide described above is a nucleotide variant of the wild type Bcl-2 gene and the corresponding gene encodes the variant of the wild type Bcl-2 protein mentioned above.

This invention further provides a viral vector having the aforementioned gene incorporated in the state capable of being expressed.

In addition, this invention provides a composition for gene therapy comprising the polynucleotide mentioned above and a pharmaceutically acceptable carrier or diluent.

As used in the specification, the terms, "Bcl-2" and "Bcl-2 protein" are used interchangeably as appropriate. In addition, the "Bcl-2" means "the wild type Bcl-2 protein" unless stated otherwise.

### Brief Description of the Drawings

Fig. 1 is a schematic structural representation of Bcl-2 showing the relative positional relation between each domain of the wild type Bcl-2 type protein and each serine residue to be substituted upon the introduction of variation.
Fig. 2 is a schematic diagram showing the scheme in which plasmids containing a Bcl-2 variant gene are constructed by the techniques including PCR, restriction enzyme digestions, and site-specific mutation according to this invention.
Fig. 3 is a bar graph comparing the wild type Bcl-2 protein to various kinds of Bcl-2 variants obtained by introducing one or more variations to said protein according to this invention with respect to the apoptosis inhibitory activity.

### Best Mode for Carrying Out the Invention

This invention will be described in detail hereafter by referring to preferred embodiments.

### (Bcl-2 Variant Protein and Bcl-2 Variant Gene)

The full amino acid sequence of Bcl-2 and the full nucleotide sequence of its corresponding Bcl-2 gene are known in the art and are shown in SEQ ID NO:1 and NO:6 in the Sequence Listing, respectively. The polypeptide of this invention is a Bcl-2 variant protein and comprises an amino acid sequence derivable from the substitution of two serines in the amino acid sequence of Bcl-2 (SEQ ID NO:1) by alanine . Preferably, the polypeptide of this invention has the apoptosis inhibitory activity substantially higher than that of the Bcl-2 protein.

The especially preferred individual variant protein is described below.

### Ser116Ala/Ser117Ala variant

It is a variant derivable from the substitution of the 116th serine by alanine and of the 117th serine by alanine in the amino acid sequence of Bcl-2, and is a polypeptide the amino acid sequence of which is set forth in SEQ ID NO:3. The gene encoding this variant is represented by the polynucleotide comprising the nucleotide sequence set forth in SEQ ID NO:8.

According to the disclosure of this specification, a Bcl-2 variant is prepared, and its apoptosis inhibitory activity is, for example, determined based on the screening panel described in the examples. When the determined apoptosis inhibitory activity is higher than that of the wild type Bcl-2, the Bcl-2 variant is within the scope of the polypeptide of this invention. According to the aforesaid disclosure and the techniques known in the art, one skilled in the art can carry out this series of manipulations without attempting undue experimentation.

The polypeptide of this invention is "substantially pure"; and as is recognized by one skilled in the protein filed, it means that when the polypeptide is produced by genetic manipulation, the polypeptide is free from contamination by proteins, nucleic acids, and other biological substances derived from the host organism. Further, it means that when the polypeptide is chemically produced by the solid phase peptide synthesis, the polypeptide is free from contamination by impurities such as the reagents used in the synthesis. The antibody is useful for the purification of the polypeptide of this invention, particularly using affinity chromatography.

### (Method of Production)

The polypeptide of this invention comprises an amino acid sequence derivable from the substitution of two serines by alanine in the amino acid sequence of Bcl-2. Once the location of serine to be substituted for is specified, the polypeptide of this invention can be produced from the Bcl-2 variant gene. For such a purpose, it is site-specific mutagenesis that is employed most commonly as the variation method of nucleotide sequence in gene. For example, M13 primer mutation, PCR and other improved methods are known. "Sambrook et al., Molecular Cloning: A Laboratory Manual (2nd Ed) Vol. 2, 1989" is referred to as a general review. If the synthetic oligonucleotide into which the desired variation has been introduced is available, the predetermined amino acid of the wild type protein to be desirably varied can be substituted by the amino acid that the variation encodes.

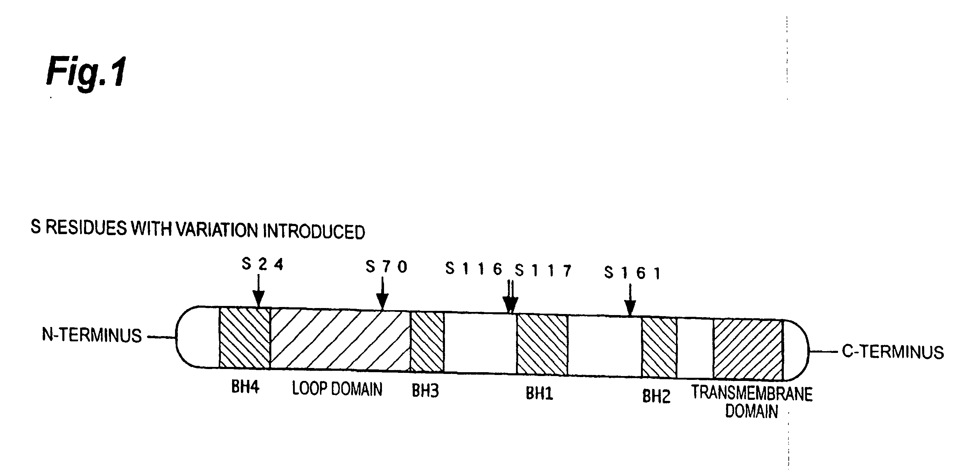

The locations of particular serine residues to be substituted for through variation as employed in the embodiments of this invention is the 116th and the 117th position in the amino acid sequence of Bcl-2. In consideration of these preferable locations, the preferable pattern of variation is a variation of such type that the 116th and the 117th serine are substituted by alanine. Fig. 1 shows the relative positional relation between these substitution (variation) locations and each domain of Bcl-2. To substitute these locations with alanine a synthetic oligonucleotide illustrated below was prepared.
Oligo-7: oligonucleotide for introducing S116A/S117A variation (SEQ ID NO:17)
5'-gtgcagctgggcggccatctcggcgaa-3'

According to the one letter designation for amino acids, "S" refers to serine, "A" to alanine in the above description.

As for the length of a synthetic oligonucleotide, it is not limited to the length adopted in the specific disclosed examples above if it is sufficient to hybridize to the wild type gene at the predetermined site to be subjected to variation.

Although a particular site-specific mutation method is described in detail in the Examples, it will be carried out by the procedure in what follows. When the synthetic oligonucleotide is treated with a kit for site-specific mutation together with a suitable plasmid into which the Bcl-2 gene is incorporated, said oligonucleotide hybridizes to the predetermined site of the gene as the template DNA under annealing conditions. This is subjected to the action of T4 DNA polymerase in the presence of T4 ligase, which results in a plasmid having the gene which contain the desired variation. The gene having undergone variation is cut out with restriction enzyme.

The thus obtained Bcl-2 variant gene is linked to a suitable vector/promoter and transferred to a host cell system, and the protein encoded by the gene is allowed to be produced, from which the peptide of this invention can be obtained by extraction and purification. These manipulations are known to one skilled in the art. The vector/promoter system to be is not particularly limited. Specifically, there are mentioned viral vectors, including MoMLV vector, HSV vector, Adenovirus vector, AAV vector, HIV vector, S1V vector, and Sendai virus vector. Usable are also a baculovirus vector capable of gene transfer to insect cells and a vector derived from tobacco mosaic virus capable of gene transfer to plant cells

As for the vectors other than those of viral origin, there may be used complexes of calcium phosphate and nucleic acid, ribosomes, cation-lipid complexes, Seidai virus liposomes, polymer carriers having polycation as the main chain and others. In addition, methods such as electroporation and gene guns may be used in gene transfer.

The promoters are not particularly limited insofar as they can allow the genes to be expressed in the host cells. Specifically, there may be mentioned: virus-derived promoters from Adenovirus, cytomegalovirus, human immunodeficiency virus, simian virus 40, Rous sarcoma virus, herpes simplex virus, murine leukemia virus, Sinbis virus, hepatitis type A virus, hepatitis type B virus, hepatitis type C virus, papilloma virus, human T cell leukemia virus, influenza virus, Japanese encephalitis virus, JC virus, parbovirus B19, poliovirus, and the like; mammal-derived promoters such as albumin, SR α, heat shock protein, and elongation factor; chimera type promoters such as CAG promoter; and promoters the expression of which can be induced by tetracycline, steroids, or the like. There may be also used promoters (such as Lac promoter) capable of expression in *E. coli* host cells.

The host cell system includes animal cells, insect cells, plant cells, *E. coli* and embryonated chicken eggs, for example.

Furthermore, with respect to the method for extracting the protein produced by the gene transferred host cell system, there are the cell homogenization method, the cell membrane lysis method utilizing a surfactant (e.g., SDS) or enzyme, ultrasonication, the freeze and thaw repetition method among others. The polypeptide of this invention can be produced by chemical synthesis using a commercially available peptide synthesizer besides gene manipulation techniques mentioned above, because their amino acid chain lengths are comparatively short. The polypeptide of this invention obtained by any of the methods may be purified according to conventional methods. Specifically, typical biochemical techniques are available for the purification which include centrifugation utilizing ultracentrifugation or density gradient centrifugation, column separation utilizing ion-exchange column or affinity column (e.g., with use of the aforementioned specific antibodies), reversed-phase column, and gel separation utilizing polyacrylamide gel.

### (Pharmaceutical Compositions)

The polypeptide that is produced and purified as described above may be combined with pharmaceutically acceptable carriers or diluents and may be formulated into pharmaceutically useful compositions. The pharmaceutical composition used in this invention comprises a therapeutically effective amount of the polypeptide mentioned above. For the suitable carriers and diluents as well as preparations containing human proteins, there are described in Remington's Pharmaceutical Sciences, for example.

The polypeptide of this invention may be formulated into a pharmaceutical composition in the form of a pharmaceutically acceptable salt. Such pharmaceutically acceptable salts include those formed with the free amino groups of protein such as those derived from hydrochloric acid, phosphoric acid, acetic acid, oxalic acid and tartaric acid and those formed with the free carboxylic acids of protein such as those derived from sodium, potassium, ammonium, calcium, ferric hydroxide, isopropylamine, triethylamine, 2-ethylaminoethanol, histidine, and procaine.

Suitable dosage forms adapted to administration for the polypeptide of this invention are not particularly limited, and preferably, they may be prepared into injectables similarly to many pharmaceutical compositions containing human proteins already for use as medicines. More specifically, the polypeptide is dissolved in a suitable solvent such as water, a physiological saline solution, and an isotonization buffer to make an injectable. Here, polyethylene glycol, glucose, various amino acids, collagen, albumen or the like may be added as protection materials for preparation. In addition, the polypeptide can be embedded into the inclusion body of ribosome and can be administered.

When the polypeptide of this invention is used in the treatment of various diseases such as those mentioned below, the dosage differs depending on the age of the subject, the body weight, the conditions, the route of administration and other factors; but the prescribing physician can readily and appropriately determine it. For example, when it is administered through parenteral administration as an injectable, the administration with a daily dose of about 0.1 µ g/kg-1000 mg/kg is preferable, and more preferably with a daily dose of about 1 µg/kg-100 mg/kg.

### (Gene Therapy)

The polynucleotide of this invention can be used as therapeutic gene (drug gene) in gene therapy. Specifically, the polynucleotide of this invention is inserted into a recombinant vector (gene transfer vector) and is delivered to the target cell, where the Bcl-2 variant gene is expressed. Then the polypeptide of this invention (i.e., a specific Bcl-2 variant) is produced in the target cell and inhibit the apoptosis of said cell.

The suitable recombinant vectors to be used for this purpose overlap the vectors capable of gene transfer to mammals, particularly human cells among those recited. Among vectors that are not disclosed above, there is mentioned a pseudo-type viral vector, for example. One representative is the pseudo-type viral vector wherein the Env protein (an envelop protein of HIV) is substituted with the VSV-G protein (an envelop protein of vesicular stomatitis virus or VSV) (Naldini L., et al., Science 272, 263 (1996)).

Likewise, the usable promoters overlap the vectors capable of gene expression in mammal cells, particularly human cells among those recited.

### (Compositions for Gene Therapy)

When the polynucleotide of this invention is used in gene therapy, it is preferably prepared as a recombinant viral vector containing a therapeutic gene designed for therapy. More specifically, the recombinant viral vector containing the polynucleotide of this invention is dissolved in a suitable solvent such as water, a physiological saline, or an isotonization buffer to prepare a composition for gene therapy.

### (Utility)

### A. Therapy

Apoptosis is involved in various disorders, and by inhibiting cell death through apoptosis (degeneration), therapeutic effects can be expected for those disorders. Accordingly, the polypeptide and polynucleotide according to this invention (which may be referred to as "Bcl-2 variant" and "Bcl-2 variant gene" hereafter) can be used for the therapy of those disorders because of their inhibitory activity. Representative disorders will be listed individually in what follows.

### 1. Alzheimer disease

Alzheimer disease is an encephalo-degenerative disorder of which progressive dysmnesia and intelligence degradation are the chief symptoms and it is known that the degeneration death of neurons causes its crisis. It has been recently understood that this cell death is induced by apoptosis. Accordingly, therapeutic effects can be expected for the treatment of Alzheimer disease by introducing the Bcl-2 variant or the Bcl-2 variant gene into the neurons.

### 2. Amyotrophic lateral sclerosis (ALS)

Amyotrophic lateral sclerosis (ALS) is an encephalo-degenerative disorder of which myodynamia depression at the distal parts of the limb, muscular atrophy, and dysphagy are the chief symptoms, and lower motor neurons are progressively and selectively damaged. Although the details of molecular biological mechanism of the crisis are unknown, it is becoming clear that apoptosis is induced by the neurons owing to gene abnormalities such as superoxide dismutase (SOD) and nerve growth nutritional factor (BDNF). Accordingly, therapeutic effects can be expected for the treatment of amyotrophic lateral sclerosis by introducing the Bcl-2 variant or the Bcl-2 variant gene into the neurons.

### 3. Nerve cell death after cerebral ischemia

Nerve cell death occurs in the hippocampus CA1 region after cerebral ischemia or cerebral infarction, which in turn proves to be the cause for dysmnesia and neurodegeneration disorder. It has recently been understood that this cell death is induced by apoptosis. Accordingly, therapeutic effects can be expected for the treatment of nerve cell death after cerebral ischemia by introducing the Bcl-2 variant or the Bcl-2 variant gene into the neurons.

### 4. Retinal degenerative disorders

In the retinal degenerative disorder, nyctalopia develops from the latter half of teens to the latter half of the 20's and then afferent narrowing of visual field progresses, leading to blindness during between 40's and 60's in many cases. It is known that these symptoms are caused by the cell degeneration of retina visual cells through apoptosis. Accordingly, therapeutic effects can be expected for the treatment of retinal degenerative disorder by introducing the Bcl-2 variant or the Bcl-2 variant gene into the retinal visual cells.

### 5. Hepatic disorders

Hepatic disorders such as hepatitis and hepatic insufficiency develop by the cell death of hepatocytes; and this cell death is known to be apoptosis. Accordingly, therapeutic effects can be expected for the treatment of hepatic disorders by introducing the Bcl-2 variant or the Bcl-2 variant gene into the hepatocystes.

### 6. Heart failure

When rat myocardial cells are cultured under anoxia in a myocardial ischemia model test, DNA laddering or an increase of Fas antigen are observed, and the cell death by apoptosis is generated. The possibility is suggested that the apoptosis of such a myocardial cell participates in heart failure such as myocardial infarction, heart failure, and hypertrophic heart. Accordingly, therapeutic effects can be expected for the treatment of heart failure by introducing the Bcl-2 variant or the Bcl-2 variant gene into the myocardial cells.

### B Other Utilities

The polypeptide and the polynucleotide according to this invention can be used for the purpose of inhibiting the cell death through apoptosis in addition to the therapy of diseases.

### 1. Production cell lines

The cell lines capable of producing useful proteins such as antibody producing cell lines (hybridomas) and viral vector producing cell lines may sometimes causes cell death because of the cytotoxicity of the protein produced while the lines are cultured for a long period of time. The production of protein can be carried out more efficiently in these cell lines by removing blood serum from the culture medium, but there is the problem that the cell death is induced in the absence of the blood serum at the same time. The major part of cell death is known to be due to apoptosis. There has been obtained the information that the production efficiency of protein increases in the strain into which the wild type Bcl-2 is incorporated (WPI97-380167/199735). It is thus expected that the production efficiency of protein increases more by the Bcl-2 variant gene transfer.

### 2. Graft cells and transplants

In the case of Parkinson disease's therapy, the technique is used that transplants the nerve cells of a dead fetus or dopamine-producing cells in the brain of a patient. Further, blood transfusion, bone marrow transplantation, and cell transplantation therapy which introduces autologous cells or the cells of others into a patient, including the ex vivo method in gene therapy, have been conducted in many instances. Graft cells typically undergo cell death by apoptosis and their long-term maintenance is difficult. Effects can be expected in organ transplantation and cell transplantation by introducing the Bcl-2 variant or the Bcl-2 variant gene into the graft cells.

### EXAMPLES

This invention will be described more concretely by way of examples; however, the invention is not to be limited by these examples.

### (Example 1) Construction of a Bcl-2 variant gene

The human wild type Bcl-2 gene was provided by S. Korsmeyer at Washington University. There were synthesized a primer having an XhoI site (which was a restriction enzyme site) just prior to the transcription start codon of the human wild type Bcl-2 (Oligo-10: SEQ ID NO:20) and a primer having an XbaI site just after the transcription stop codon (Oligo-11: SEQ ID NO:21), respectively. PCR was performed in the procedure described below.

Human Bcl-2 gene, 1 µl, dNTP (2nM: Promega Corporation), 5 µl, Oligo-10 (50 µM), 1 µl, Oligo-11 (50 µM), 1 µl, polymerase (2-3 units: Promega Corporation), 1 µl pfu, dH₂O, 31 µl, were mixed to a total of 50 µl. After allowing this mixed solution at 95 ° c for 5 minutes, reaction was done in two cycles of which one cycle consisted of (94 ° c for 30 seconds), (55 ° c for 30 seconds), and (72 ° c for 60 seconds) and further, the reaction was repeated in 20 cycles of which one cycle consisted of (94 ° c for 30 seconds), (58 ° c for 30 seconds), and (72 ° c for 60 seconds). The obtained PCR product was incorporated in PBS-SK (+) (STRATAGENE) at its XhoI-Xbal site to prepare pBS-bcl2.

Employing pBS-bcl2 and one antisense oligonucleotide for variation introduction that was chemically synthesized (Oligo -7: SEQ ID NO:17), a variation was introduced at two positions of the Bcl-2 gene with an Altered Sites II-Exl *in vitro* Mutagenesis System (Promega Corporation) according to the procedure described below.

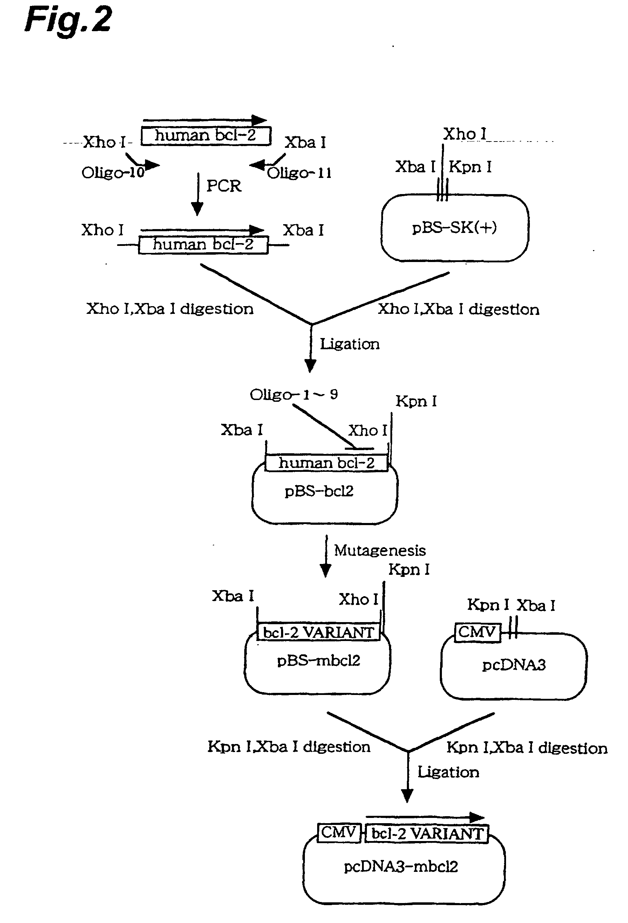

pBS-bcl2 (200ng), 2 µl, Ampicillin Repair Oligonucleotide (0.25 µM: kit accessory available from Promega Corporation), 1 µl, Tetracycline Knockout Oligonucleotide (0.25 µM: kit accessory available from Promega Corporation.), 1 µl, an oligonucleotide for variation introduction (1.25 µM), 1 µl, Annealing 10Xbuffer (kit accessory available from Promega Corporation.), 2 µl, dH₂O, 13 µl, were mixed to a total of total 20 µl. After allowing this mixed solution at 95 ° c for 5 minutes and then at 75 ° c for 6 minutes, temperature was lowered to 45 ° c at a rate of 1 ° c per second. Subsequently, reaction was stopped at 37 ° c. To this reaction solution were mixed, Synthesis 10Xbuffer (kit accessory available from Promega Corporation.), 3 µl, T4 DNA polymerase (5-10 units: kit accessory available from Promega Corporation.), 1 µl, T4 DNA ligase (1-3 units: kit accessory available from Promega Corporation.), dH₂O, 5 µl, making a total of 30 µl. This mixed solution was further allowed to react at 37 ° c for 90 minutes, whereby there was obtained a Bcl-2 variant DNA corresponding to the antisense oligonucleotide of the variation type ( Oligo -7). The Bcl-2 variant DNA was incorporated in pcDNA3 (Invitrogen Corporation) carrying a cytomegalovirus promoter capable of transcription in animal cells and finally, a viral expression vector having the Bcl-2 variant gene incorporated were constructed. See Fig. 2.

### (Example 2) Variation gene transfer to cells

The calcium phosphate method was used in the procedure described below to conduct gene transfer into a BHK cell (syrian hamster kidney origin) with the following genes: the Bcl-2 variant gene constructed in Example 1, the wild type Bcl-2 gene (as positive control), and pcDNA3 (as negative control).

BHK cells were seeded on a cover slip (18mm: Matsunami Glass Co. Ltd.) to provide a level of 0.8-1.6x10⁴. Culturing was done under the conditions of 37 ° c and 5% CO₂ overnight. After culturing, the culture medium was changed once and the culturing was done under the conditions of 37 ° c and 5% CO₂ for additional 2-4 hours.

The gene/calcium phosphate solution was prepared according to the following method. To 30 µl of a calcium chloride solution (0.2 M.CaCl₂, 50 mM Hepes), were added the wild type Bcl-2, 4 µl, pcDNA3 (GFP), and the Bcl-2 variant gene (each 2 µg). Further, to the solution was added dropwise 30 µl of 2xHBS (50 mM Hepes, 280 mM NaCl, 10 mM KCl, 1.5mM Na₂HPO₄ · 12H₂O, 12 mM glucose, 0.1xPBS) under shaking. It was left at room temperature for 15 minutes, and 350 µl of medium was added last to prepare gene/calcium phosphate solutions. The prepared gene/calcium phosphate solution was added to the cells. After culturing for 4 hours, the cells were washed and the culture medium was changed. Further, after 16 hours' culturing, 4 mM of Dexamethazone (Sigma Company Ltd.) was added to the cells, inducing apoptosis. Furthermore, the cells were fixed with 3% formaldehyde after 8 hours' culturing and were used in the test that follows.

### (Example 3) Comparison of apoptosis inhibitory effects by nuclear staining

The cells obtained in Example 2 and fixed with 3% formaldehyde, for which apoptosis was induced, were stained with 10mg/ml of Hoechst33258 (Molecular Probe Inc.) for one minute and were observed under a microscope.

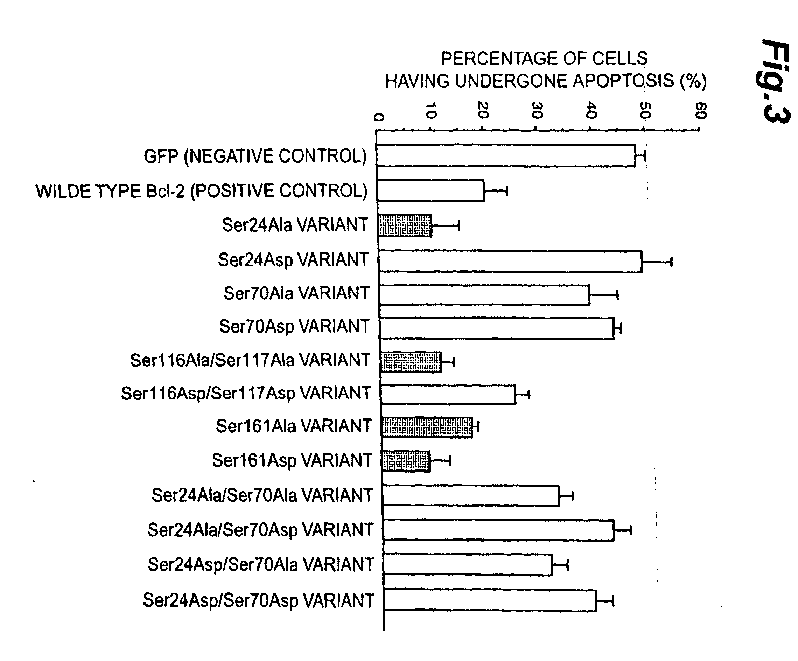

For the determination of apoptosis, the followings were observed and employed as indicators: (1) the distinct contraction of cytoskeleton; (2) the concentration of nucleus and increased staining; (3) round-up and blebbing of cell; (4) fragmentation of nucleus. (Wyllie et al., Int. Rev. Cytol. 68, 251 (1980).) If any one of (1) to (4) is applicable, the cell will be judged to be positive, and the number of cells is shown as a percentage of 1500 cells. The results obtained are shown in Fig. 3. The apoptosis inhibitory activity was significantly enhanced in the Ser116Ala/Ser117Ala variant when compared with the wild type Bcl-2 which was positive control.

### Industrial Applicability

As described above, the polynucleotide and the polypeptide according to this invention are variants of the Bcl-2 gene, which is a protooncogene , and Bcl-2 variant protein possessing apoptosis inhibitory activity, respectively; and said protein is provided with significantly enhanced apoptosis inhibitory activity as compared with the wild type Bcl-2 protein.

Accordingly, the polypeptide of this invention is useful for the treatment of various disorders for which the apoptosis of cells is responsible.

Furthermore, because the polynucleotide of this invention can be the gene encoding the polypeptide possessing the apoptosis inhibitory activity, it will be useful for the treatment of various disorders for which the apoptosis of cells is responsible when applied in gene therapy.

## Claims

1. A polypeptide comprising the amino acid sequence set forth in SEQ ID NO: 3.

2. A polynucleotide encoding the polypeptide according to claim 1.

3. A polynucleotide comprising the nucleotide sequence set forth in SEQ ID NO: 8.

4. A pharmaceutical composition comprising the polypeptide according to claim 1 and a pharmaceutically acceptable carrier or diluent.

5. A viral vector having the gene according to claim 2 incorporated therein in a form capable of being expressed.

6. A composition for gene therapy comprising the polynucleotide according to claim 3 and a pharmaceutically acceptable carrier or diluent.

## Patentansprüche

1. Ein Polypeptid, das die Aminosäuresequenz, die in SEQ ID NO: 3 gezeigt wird, umfasst.

2. Ein Polynucleotid, das das Polypeptid gemäß Anspruch 1 kodiert.

3. Ein Polynucleotid, das die Nucleotidsequenz, die in SEQ ID NO: 8 gezeigt wird, umfasst.

4. Eine pharmazeutische Zusammensetzung, die das Polypeptid gemäß Anspruch 1 und ein pharmazeutisch verträgliches Trägermittel oder Verdünnungsmittel umfasst.

5. Ein viraler Vektor mit dem Gen gemäß Anspruch 2, das darin in einer Form vorhanden ist, die in der Lage ist, exprimiert zu werden.

6. Eine Zusammensetzung zur Gentherapie, die das Polynucleotid gemäß Anspruch 3 und ein pharmazeutisch verträgliches Trägermittel oder Verdünnungsmittel umfasst.

## Revendications

1. Polypeptide comprenant la séquence en acides aminés indiquée dans SEQ ID NO:3.

2. Polynucléotide codant pour le polypeptide selon la revendication 1.

3. Polynucléotide comprenant la séquence nucléotidique indiquée dans SEQ ID NO:8

4. Composition pharmaceutique comprenant le polypeptide selon la revendication 1 et un véhicule ou diluant pharmaceutiquement acceptable.

5. Vecteur viral portant le gène selon la revendication 2 incorporé dans celui-ci sous une forme capable d'être exprimée.

6. Composition pour la thérapie génique comprenant le polynucléotide selon la revendication 3 et un véhicule ou diluant pharmaceutiquement acceptable.
